# EUROPEAN PATENT SPECIFICATION

(11) **EP 2 764 366 B1**
(45) Date of publication and mention of the grant of the patent: **20.07.2016**
(21) Application number: 12719436.3
(22) Date of filing: 02.04.2012
(51) Int. Cl.: G01N 33/68

(54) **PROGNOSTIC METHOD FOR DIABETES**
PROGNOSTISCHES VERFAHREN FÜR DIABETES
PROCÉDÉ DE PRONOSTIC POUR LE DIABÈTE

(30) Priority: 05.10.2011 GB 201117172
(43) Date of publication of application: 13.08.2014
(73) Proprietor: The Binding Site Group Limited, Birmingham, West Midlands B15 1QT (GB)
(72) Inventor: HUTCHISON, Colin, Birmingham West Midlands B15 1QT (GB); HARDING, Stephen, Birmingham West Midlands B15 1QT (GB); HUGHES, Richard Geir, Birmingham West Midlands B15 1QT (GB)
(74) Representative: Elsy, David
(86) International application number: PCT/GB2012/050734
(87) International publication number: WO 2013/050731

(56) References cited:
- WO-A1-2011/107965
- US-A1- 2009 023 165
- DATABASE BIOSIS [Online] BIOSCIENCES INFORMATION SERVICE, PHILADELPHIA, PA, US; 1997, IEKI YASUHIKO ET AL: "Analysis of urinary kappa light chain as a predictor of early diabetic nephropathy: Five-year follow-up study", XP002676715, Database accession no. PREV199799763130 & JOURNAL OF THE JAPAN DIABETES SOCIETY, vol. 40, no. 7, 1997, pages 409-416, ISSN: 0021-437X
- HUTCHISON C A ET AL: "Quantitative assessment of serum and urinary polyclonal free light chains in patients with type II diabetes: An early marker of diabetic kidney disease?", EXPERT OPINION ON THERAPEUTIC TARGETS 200806 GB LNKD- DOI:10.1517/14728222.12.6.667, vol. 12, no. 6, June 2008 (2008-06), pages 667-676, XP008132516, ISSN: 1472-8222 cited in the application
- COLIN A HUTCHISON ET AL: "Quantitative assessment of serum and urinary polyclonal free light chains in patients with chronic kidney disease", CLINICAL JOURNAL OF THE AMERICAN SOCIETY OF NEPHROLOGY : CJASN,, vol. 3, no. 6, 1 November 2008 (2008-11-01), pages 1684-1690, XP002652096, ISSN: 1555-905X, DOI: 10.2215/CJN.02290508 [retrieved on 2008-10-22] cited in the application
- S.B. HASSAN ET AL: "Urinary [kappa] and [lambda] immunoglobulin light chains in normoalbuminuric type 2 diabetes mellitus patients", JOURNAL OF CLINICAL LABORATORY ANALYSIS, vol. 25, no. 4, 1 January 2011 (2011-01-01), pages 229-232, XP55028252, ISSN: 0887-8013, DOI: 10.1002/jcla.20463
- TAKANARI NAKANO ET AL: "Free immunoglobulin light chain: Its biology and implications in diseases", CLINICA CHIMICA ACTA, vol. 412, no. 11-12, 1 May 2011 (2011-05-01), pages 843-849, XP55028255, ISSN: 0009-8981, DOI: 10.1016/j.cca.2011.03.007
- KATZMANN JERRY A ET AL: "Serum reference intervals and diagnostic ranges for free kappa and free lambda immunoglobulin light chains: Relative sensitivity for detection of monoclonal light chains", CLINICAL CHEMISTRY, AMERICAN ASSOCIATION FOR CLINICAL CHEMISTRY, WASHINGTON, DC, vol. 48, no. 9, 1 September 2002 (2002-09-01), pages 1437-1444, XP002378160, ISSN: 0009-9147
- L Assi ET AL: "Total serum free light chains are an important prognostic marker in patients with Type II Diabetes", J Am Soc Nephrol 22(s):TH-PO507a, November 2011 (2011-11), XP55028259, Retrieved from the Internet: URL:http://www.asn-online.org/education_an d_meetings/kidneyweek/archives/ [retrieved on 2012-05-29]

## Description

The field of the invention relates to methods of determining long-term prognosis in subjects with diabetes by measuring free light chains (FLC).

Diabetes mellitus, diabetes, is a group of diseases in which a subject has high blood sugar levels, often because the body does not produce enough insulin or because cells do not respond to the insulin that is produced by the subject. Type 1 diabetes results from the subject's failure to produce insulin. Type 2 (type-II, non-insulin or adult-onset diabetes) results from insulin resistance where a subject's cells fail to respond normally to insulin, sometimes combined with absolute insulin deficiency. Other types of diabetes include genetic or congenital related diabetes, steroid diabetes and gestational diabetes.

Diabetes is associated with a number of disorders including chronic kidney disease (CKD), cardiovascular disease (CVD) and retinal damage.

The Applicants have for many years studied free light chains as a way of assaying for a wide-range of monoclonal gammopathies in patients. The use of such free light chains in diagnosis is reviewed in detail in the book "Serum Free Light Chain Analysis, Sixth Edition (2010) A.R. Bradwell et al, ISBN 9780704427969".

Antibodies comprise heavy chains and light chains. They usually have a two-fold symmetry and are composed of two identical heavy chains and two identical light chains, each containing variable and constant region domains. The variable domains of each light-chain/heavy-chain pair combine to form an antigen-binding site, so that both chains contribute to the antigen-binding specificity of the antibody molecule. Light chains are of two types, κ and λ and any given antibody molecule is produced with either light chain but never both. There are approximately twice as many κ as λ molecules produced in humans, but this is different in some mammals. Usually the light chains are attached to heavy chains. However, some unattached "free light chains" are detectable in the serum or urine of individuals. Free light chains may be specifically identified by raising antibodies against the surface of the free light chain that is normally hidden by the binding of the light chain to the heavy chain. In free light chains (FLC) this surface is exposed, allowing it to be detected immunologically. Commercially available kits for the detection of κ or λ free light chains include, for example, "Freelite™", manufactured by The Binding Site Limited, Birmingham, United Kingdom. The Applicants have previously identified that determining the amount of free κ/free λ ratios, aids the diagnosis of monoclonal gammopathies in patients. It has been used, for example, as an aid in the diagnosis of intact immunoglobulin multiple myeloma (MM), light chain MM, non-secretory MM, AL amyloidosis, light chain deposition disease, smouldering MM, plasmacytoma and MGUS (monoclonal gammopathies of undetermined significance). Detection of FLC has also been used, for example, as an aid to the diagnosis of other B-cell dyscrasia and indeed as an alternative to urinary Bence Jones protein analysis for the diagnosis of monoclonal gammopathies in general.

Conventionally, an increase in one of the λ or κ light chains and a consequently abnormal ratio is looked for. For example, multiple myelomas result from the monoclonal multiplication of a malignant plasma cell, resulting in an increase in a single type of cell producing a single type of immunoglobulin. This results in an increase in the amount of free light chain, either λ or κ, observed within an individual. This increase in concentration may be determined, and usually the ratio of the free κ to free λ is determined and compared with the normal range. This aids in the diagnosis of monoclonal disease. Moreover, the free light chain assays may also be used for the following of treatment of the disease in patients. Prognosis of, for example, patients after treatment for AL amyloidosis may be carried out.

Katzmann et al (Clin. Chem. (2002); 48(9): 1437-1944) discuss serum reference intervals and diagnostic ranges for free κ and free λ immunoglobulins in the diagnosis of monoclonal gammopathies. Individuals from 21-90 years of age were studied by immunoassay and compared to results obtained by immunofixation to optimise the immunoassay for the detection of monoclonal free light chains (FLC) in individuals with B-cell dyscrasia.
The amount of κ and λ FLC and the κ/λ ratios were recorded allowing a reference interval to be determined for the detection of B-cell dyscrasias.

Attempts have been made to investigate whether chronic kidney disease can be correlated with FLC production in diabetics.

Hutchinson C. A. et al (Expert. Opin Ther. Targets (2008) 12(6) 667-676) describes the quantitative assessment of serum and polyclonal free light chains in patients with type II diabetes. The study showed that both κ and λ serum FLC were significantly raised in diabetic patients. Even patients with normal renal function had elevated serum FLCs (sFLC). There was some correlation between sFLC and cystatin C but not correlation between HbA1C (glycated haemoglobin) levels and sFLC.

Most of the patients had mild or no impairment of renal function. The paper noted that both κ and λ were elevated in patients even if they had normal renal functions. sFLC was elevated in many patients independently of kidney function. The paper speculates that FLC might be a marker of inflammation in diabetes. The paper describes sFLC as correlating to chronic kidney disease (CKD), with patients with CKD also having elevated FLC. It speculates that FLC in urine or serum may be a predictor of the outcome of kidney disease. However, it notes that the high percentage of diabetic patients with abnormal FLC meant that FLC alone was not a useful predictor of outcome, but may need to be combined with other markers. These would be useful in the management of diabetic kidney disease. See also Hutchison et. al., Clin J Am Soc Nephrol 3, 1684-1690, 2008.

The Applicant has now studied survival data in patients with diabetes and identified that FLC concentration correlates strongly to overall long-term survival in diabetic subjects.

An advantage to being able to identify subjects with shorter survival prospects, is that they can be identified and directed towards specialist diabetes treatment centers for intensive monitoring and management, often before chronic symptoms such as chronic kidney disease, cardiovascular disease or retinal damage become apparent in the subject. It is expected that such early intervention will improve the long-term prognosis of the subject compared to those that had they not been treated earlier.

The invention provides a method of determining a long-term prognosis in a subject identified as having diabetes, comprising detecting a total (i.e. combined λ and κ) amount of free light chains (FLC) in a sample from the subject, wherein the total amount of FLC in the sample is compared to a predetermined normal amount and a higher amount of FLC is associated with increased risk of a cardiovascular disease event or cardiovascular disease related death, wherein the sample is a sample of blood, serum or plasma and the subject does not present with symptoms of a B cell disease or chronic kidney disease (CKD), and the long term prognosis is at least 200 days from when the sample is taken.

Typically the subject has been previously identified as having diabetes.

The method provides for an early indication of the increased risk of cardiovascular disease, small vessel disease, retinopathy, peripheral neuropathy and/or autonomic neuropathy in the subject.

Analysis of serum samples from patients with Type II diabetes showed combined (λ and κ FLC) in serum was independently associated with adverse cardiovascular disease (CVD) outcomes.

The invention provides a method of identifying a risk of developing one or more conditions selected from cardiovascular disease such as small vessel disease; retinopathy; peripheral neuropathy; and autonomic neuropathy in a diabetic subject comprising detecting an amount of FLC, (such as total FLC) in a sample from the subject, wherein a higher amount of FLC is associated with an increased risk of developing the, or each, condition.

The subject may have any form of diabetes. Typically the subject has type 2 diabetes.

Total FLC is determined in the sample from the subject. Further examples describe herein that the amount of κ FLC or λ FLC may be determined in the sample.

Further examples describe herein that the sample may be any tissue sample or other sample from the subject. Typically the sample is blood, serum or plasma from the subject.

The term "decreased probability of long-term survival" means that the subject has an increased risk of death compared to a diabetic subject with lower amounts of FLC present. An increase or decrease in survival has been detected over a period of as little as 200 days. Hence long-term typically means at least 200, at least 500 or more days from when the sample was taken.

Small vessel disease is a disease which affects the small arteries of the body. Such small arteries normally provide blood to the heart, brain and other body organs. This is distinct to, but often co-existing with, coronary heart disease which affects the large arteries to the heart.

Common symptoms of small vessel disease are angina, leg ulcers, leg pain, acute or progressive loss of neurological function (transient ischaemic attacks) and vascular dementia.

Retinopathy affects up to 80% of patients who have had diabetes for 10 years or more. Many of these cases can be reduced by vigilant treatment and early monitoring of the eyes. The ability to help predict retinopathy would assist in reducing eye damage in diabetic subjects.

Peripheral and autonomic neuropathy are disorders often associated with diabetes and can result in a very high morbidity and thus requiring frequent hospital admissions for the patient. They may be associated with small blood vessel damage.

The method typically compares the total amount of FLC in the sample to a predetermined normal amount. There is some variation of normal FLC produced in healthy patients from population to population or from source of sample to source of sample. However, typically the term "higher amount" means above 48, 50 mg/L total FLC in serum or above 57 mg/L, above 60 mg/L or above 65 mg/L. The typical normal values for kappa and lambda above which is considered significant are 19.4 mg/L for kappa and 26.3 mg/L for lambda.

Also described herein is that the results of the method may be used in combination with one or more additional markers or indicators to further confirm the prognosis. For example, age (above 65) also appears to be a factor. Other variables which may also be considered include eGFR (estimated Glomerolar Filtration Rate), which may be calculated based on creatinine secretion, Body Mass Index (BMI), HBA1C (glycated haemoglobin), plasma glucose at diagnosis.

Total free light chain means the total amount of free kappa plus free lambda light chains in a sample.

Typically the subject does not necessarily have symptoms of a B-cell disease and/or chronic kidney disease. Such symptoms may include recurrent infections, bone pain and fatigue. Such a B-cell disease is preferably not a myeloma, (such as intact immunoglobulin myeloma, light chain myeloma, non-secretory myeloma), an MGUS, AL amyloidosis, Waldenström's macroglobulinaemia, Hodgkin's lymphoma, follicular centre cell lymphoma, chronic lymphocytic leukaemia, mantle cell lymphoma, pre-B cell leukaemia or acute lymphoblastic leukaemia. Moreover, the individual typically does not have reduced bone marrow function. The individual typically does not have an abnormal κ: λ FLC ratio, typically found in many such diseases.
Common symptoms of chronic kidney disease include tiredness, nausea, itching and peripheral oedema.
The term "total free light chains" means the amount of κ and λ free light chains in the sample from the subject.

The sample is typically a sample of serum from the subject. However, whole blood or plasma may be used. Also described herein are that urine or other samples of tissue or fluids may also potentially be utilised.
Typically the FLC, such as total FLC, is determined by immunoassay, such as ELISA assays or utilising fluorescently labelled beads, such as Luminex™ beads.
Sandwich assays, for example, use antibodies to detect specific antigens. One or more of the antibodies used in the assay may be labelled with an enzyme capable of converting a substrate into a detectable analyte. Such enzymes include horseradish peroxidase, alkaline phosphatase and other enzymes known in the art. Alternatively, other detectable tags or labels may be used instead of, or together with, the enzymes. These include radioisotopes, a wide range of coloured and fluorescent labels known in the art, including fluorescein, Alexa fluor, Oregon Green, BODIPY, rhodamine red, Cascade Blue, Marina Blue, Pacific Blue, Cascade Yellow, gold; and conjugates such as biotin (available from, for example, Invitrogen Ltd, United Kingdom). Dye sols, chemiluminescent labels, metallic sols or coloured latex may also be used. One or more of these labels may be used in the ELISA assays according to the various inventions described herein or alternatively in the other assays, labelled antibodies or kits described herein.
The construction of sandwich-type assays is itself well known in the art. For example, a "capture antibody" specific for the FLC is immobilised on a substrate. The "capture antibody" may be immobilised onto the substrate by methods which are well known in the art. FLC in the sample are bound by the "capture antibody" which binds the FLC to the substrate via the "capture antibody".

Unbound immunoglobulins may be washed away.

In ELISA or sandwich assays the presence of bound immunoglobulins may be determined by using a labeled "detecting antibody" specific to a different part of the FLC of interest than the binding antibody.

Flow cytometry may be used to detect the binding of the FLC of interest. This technique is well known in the art for, e.g. cell sorting. However, it can also be used to detect labeled particles, such as beads, and to measure their size. Numerous text books describe flow cytometry, such as Practical Flow Cytometry, 3rd Ed. (1994), H. Shapiro, Alan R. Liss, New York, and Flow Cytometry, First Principles (2nd Ed.) 2001, A.L. Given, Wiley Liss.

One of the binding antibodies, such as the antibody specific for FLC, is bound to a bead, such as a polystyrene or latex bead. The beads are mixed with the sample and the second detecting antibody. The detecting antibody is preferably labeled with a detectable label, which binds the FLC to be detected in the sample. This results in a labeled bead when the FLC to be assayed is present.

Other antibodies specific for other analytes described herein may also be used to allow the detection of those analytes.

Labeled beads may then be detected via flow cytometry. Different labels, such as different fluorescent labels may be used for, for example, the anti- free λ and anti- free κ antibodies. Also described herein are that assays such as generally known liver function tests may be used in combination with this method.

Alternatively, or additionally, different sized beads may be used for different antibodies, for example for different marker specific antibodies. Flow cytometry can distinguish between different sized beads and hence can rapidly determine the amount of each FLC or other analyte in a sample.

An alternative method uses the antibodies bound to, for example, fluorescently labeled beads such as commercially available Luminex™ beads. Different beads are used with different antibodies. Different beads are labeled with different fluorophore mixtures, thus allowing different analytes to be determined by the fluorescent wavelength. Luminex beads are available from Luminex Corporation, Austin, Texas, United States of America.

Preferably the assay used is a nephelometric or turbidimetric method. Nephelometric and turbidimetric assays for the detection of λ - or κ - FLC are generally known in the art, but not for total FLC assays. They have the best level of sensitivity for the assay. λ and κ FLC concentrations may be separately determined or a single assay for total FLC arrived at. Such an assay contains anti-K and anti-λ FLC antibodies typically at a 60:40 ratio, but other ratios, such as 50:50 may be used.

Antibodies may also be raised against a mixture of free λ and free κ light chains.

The amount of total FLC is compared to a standard, predetermined value to determine whether the total amount is higher or lower than a normal value. Patients with amounts above 50 mg/L FLC in serum, for example, has been shown to have significantly reduced survival.

Historically, assay kits have been produced for measurement of kappa and lambda FLC separately, to allow the calculation of a ratio. They have been conventionally used in individuals already exhibiting disease symptoms.

Preferably the assay is capable of determining FLC, for example total FLC, in the sample for example from approximately 1 mg/L to 100 mg/L, or 1 mg/L - 80 mg/L. This is expected to detect the serum FLC concentrations in the vast majority of individuals without the requirement for re-assaying samples at a different dilution.

The method may comprise detecting the amount of total free light chain in the sample utilising an immunoassay, for example, by utilising a mixture of anti-free κ light chain and anti-free λ light chain antibodies or fragments thereof. Such antibodies may be in a ratio of 50:50 anti-K: anti-λ antibodies. Antibodies, or fragments, bound to FLC may be detected directly by using labelled antibodies or fragments, or indirectly using labelled antibodies against the anti-free λ or anti-free κ antibodies.

The antibodies may be polyclonal or monoclonal. Polyclonal may be used because they allow for some variability between light chains of the same type to be detected as they are raised against different parts of the same chain. The production of polyclonal antibodies is described, for example in WO97/17372.

Assay kits for FLC, for example for use in the methods of the invention are also described herein. The kits may detect the total amount FLC in a sample. They may be provided in combination with instructions for use in the methods of the invention.

The assay kits may be adapted to detect an amount of total free light chain (FLC) in a sample below 25 mg/L, most preferably, below 20 mg/L or about, 10 mg/L, below 5 mg/L or 4 mg/L. The calibrator material typically measures the range 1-100mg/L. The assay kit may be, for example, a nephelometric assay kit. Preferably the kit is an immunoassay kit comprising one or more antibodies against FLC. Typically the kit comprises a mixture of anti-K and anti-λ FLC antibodies. Typically a mixture of 50:50 anti-free κ and anti-free λ antibodies are used. The kit may be adapted to detect an amount of 1 - 100 mg/L, or preferably 1 - 80 mg/L total free light chain in a sample.

Fragment of antibodies, such as (Fab)₂ or Fab antibodies, which are capable of binding FLC may also be used.

The antibodies or fragments may be labelled, for example with a label as described above. Labelled anti-immunoglobulin binding antibodies or fragments thereof are also described herein to detect anti-free λ or anti-free κ bound to FLC.

Also described herein are kits which may comprise calibrator fluids to allow the assay to be calibrated at the ranges indicated. The calibrator fluids preferably contain predetermined concentrations of FLC, for example 100mg/L to 1mg/L, below 25 mg/L, below 20 mg/L, below 10 mg/L, below 5 mg/L or to 1 mg/L. The kit may also be adapted by optimising the amount of antibody and "blocking" protein coated onto the latex particles and, for example, by optimising concentrations of supplementary reagents such as polyethylene glycol (PEG) concentrations.

The kit may comprise, for example, a plurality of standard controls for the FLC. The standard controls may be used to validate a standard curve for the concentrations of the FLC or other components to be produced. Such standard controls confirm that the previously calibrated standard curves are valid for the reagents and conditions being used. They are typically used at substantially the same time as the assays of samples from subjects. The standards may comprise one or more standards below 20 mg/L for FLC, more preferably below 15 mg/L, below approximately 10 mg/L or below 5 mg/L, in order to allow the assay to detect the lower concentrations of free light chain.

Also described herein is an assay kit which may be nephelometric or turbidimetric kit. It may be an ELISA, flow cytometry, fluorescent, chemiluminescent or bead-type assay or dipstick. Such assays are generally known in the art.

Also described herein is an assay kit which may also comprise instructions to be used in the method according to the invention. The instructions may comprise an indication of the concentration of total free light chain considered to be a normal value, below which, or indeed above which, shows an indication of either increased or decreased likelihood of the survival being present, for example. Such concentrations may be as defined above.
(3.8), BMI: 28 (7), eGFR: 76.8 (19.3), HBA1C: 6.7 (2.4), triglycerides: 2.2 (1.6) and total FLCs: 41.10 (21.5).

The prognostic value of each variable was assessed using Kaplan Meier and Cox-regression analysis. A total of 164 (80 males and 84 females) had elevated FLC levels (normal range: 9-48 mg/L). By 4.5years 17/525 (3.2%) patients had died. Total FLC levels were significantly higher in patients who had died (52.8 mg/L) compared with those who were still alive (40.9 mg/L) (p=0.026). Patients with elevated FLC levels (>50 mg/L) had a significantly shorter OS than patients with FLCs <50 mg/L (p=0.013).

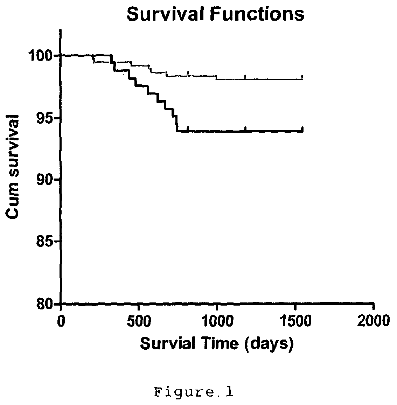

The following variables were included as part of the univariate analysis: systolic BP, diastolic BP, age, gender, creatinine, smoking status, total cholesterol, HDL, LDL, ACR, BMI, eGFR, HBA1C, triglycerides, hsCRP and total FLCs. Only raised FLCs (>50mg/L) (HR=3.2, p=0.02), age>65 yrs (HR=3.24, p=0.021), ACR (HR=3.33, p=0.016) and eGFR (HR=0.97, 95% CI=0.93-0.99, p=0.036) were identified to be prognostic of survival. When eGFR was calculated using a categorical cutoff (quartile for the population), the HR was 3.02 (p=0.06). Of these, only FLC>50 mg/L were shown to be independently associated with OS in the multivariate analysis (HR=5.21; p=0.015). The Kaplan Meier plot for FLC is shown in Figure 1.

To conclude, elevated serum FLCs (>50 mg/L) were independently associated with a shorter overall survival in patients with type II diabetes. The causes of deaths where recorded, included a variety of difference causes, including cardiovascular related deaths.

The early identification of patients at risk of early death allows those at risk to be directed to specific treatment before other symptoms become apparent, and is expected to improve long-term survival in those patients with improved management of the diabetes.

Total (or "combined") serum free light chains predict poor cardiovascular outcomes in type II diabetes.

### Background/Introduction

Type II diabetes is associated with an increased risk of cardiovascular disease (CVD), with heart disease or stroke representing the primary causes of death in diabetic patients. The purpose of this study was to determine if serum free light chains (FLC) predicted adverse CVD outcomes in diabetic patients.

### Methods

Stored serum samples from 352 South Asian type II diabetic patients were retrieved and assessed retrospectively for serum FLC, C reactive protein (CRP) and cystatin C. The results were analysed in relation to baseline measurements of standard clinical variables including blood pressure (Diastolic blood pressure (DBP, mmHg), systolic blood pressure (SBP, mmHg) and mean arterial pressure (MAP, mmHg)), lipid levels (High density lipoprotein (HDL) cholesterol (mmol/L), low density lipoprotein (LDL) cholesterol (mmol/L), total cholesterol (mmol/L) and triglyceride (mmol/L)), renal function measurements (Albumin creatinine ratio (ACR), creatinine (µmol/L) and estimated glomerular filtration rate (eGFR, ml/min/1.73m³), glycated haemoglobin (HbA1C, %), age (years) and body mass index (BMI). CVD events (including myocardial infarction, cerebrovascular accident, heart failure and coronary artery bypass graft) or CVD deaths over 2 years were examined. Each variable was categorized on clinical reference ranges, and additionally by using Receiver operating characteristic (ROC) curve analysis to determine the optimal cut off value. The association between variables and CVD outcomes were analysed by binary logistic regression analysis using SPSS version 19.

### Results

**Table 1**

| | **Median** | **IQR** |
|---|---|---|
| Combined FLC (cFLC summated FLC kappa and FLC lambda, mg/L) | 43.3 | 21.5 |
| High sensitivity C reactive protein (hsCRP, mg/L) | 3.68 | 5.3 |
| Age (years) | 55 | 19 |
| Albumin creatinine ratio | 0.9 | 4.3 |
| Creatinine (µmol/L) | 84.5 | 24.3 |
| Estimated glomerular filtration rate (eGFR, ml/min/1.73m³) | 75.6 | 18.6 |
| Body mass index (BMI) | 28 | 6 |
| Diastolic blood pressure (DBP, mmHg) | 82 | 14 |
| Systolic blood pressure (SBP, mmHg) | 133 | 28 |
| Mean arterial pressure (MAP, mmHg) | 99.3 | 16.8 |
| High density lipoprotein (HDL) cholesterol (mmol/L) | 1.1 | 0.3 |
| Low density lipoprotein (LDL) cholesterol (mmol/L) | 2.3 | 1.1 |
| Total cholesterol (mmol/L) | 4.6 | 1.5 |
| Triglyceride (mmol/L) | 2.3 | 1.8 |
| Glycated heamoglobin (HbA1C, %) | 8.1 | 2.7 |

| | | |
|---|---|---|
| Baseline patient characteristics. IQR = interquartile range. | | |

During follow up, 23/352 patients suffered a CVD event and there were 7 CVD related deaths. One patient who suffered an event subsequently died, giving a total of 29/352 patients who had an adverse CVD outcome (CVD event or CVD related death). The results of logistic regression analysis are shown in tables 2-5.

**Table 2**

| | **Cut off** | **Hazard ratio** | **p** |
|---|---|---|---|
| cFLC | >50mg/L | 2.450 | **0**.**022** |
| Age | >60 | 2.661 | **0**.**014** |
| Albumin creatinine ratio | >2.5 M >3.5 F | 3.077 | **0**.**005** |
| HbAlc | >6.5% | 2.080 | **0**.**094** |
| MAP | 70-110mmHg) | 3.225 | **0**.**003** |
| CHD history | | 2.943 | **0**.**010** |

Univariate logistic regression analysis using the indicated clinical reference range cut offs. Only the variables that predicted adverse CHD outcomes at a significance of p<0.1 are shown, and were selected for inclusion in a multivariate analysis.

**Table 3**

| | **Cut off** | **Hazard ratio** | **p** |
|---|---|---|---|
| Albumin creatinine ratio | >2.5 M >3.5 F | 2.635 | **0**.**019** |
| MAP | 70-110mmHg) | 3.415 | **0**.**003** |

Multivariate logistic regression analysis using the clinical reference range cut offs. Abnormal albumin/creatinine ratio and MAP were independently associated with adverse CHD outcomes.

**Table 4**

| | **Cut off** | **HR** | **p** |
|---|---|---|---|
| Albumin creatinine ratio | >2.5 M >3.5 F | 3.077 | **0**.**005** |
| MAP | 70-110mmHg) | 3.225 | **0**.**003** |
| cFLC | ≥57.2mg/L | 3.164 | **0**.**004** |
| hsCRP | >7.631mg/L | 2.550 | **0**.**031** |
| Age | >62 | 2.920 | **0**.**007** |
| HbAlc | >9.2% | 2.224 | **0**.**048** |
| SBP | >155mmHg | 3.516 | **0**.**002** |
| CHD history | | 2.943 | **0**.**010** |

Univariate logistic regression analysis using optimal cut offs, as indicated. Variables that predicted adverse CHD outcomes at a significance of p<0.1 are shown, and were selected for inclusion in a multivariate analysis.

**Table 5**

| | **Cut off** | **HR** | **p** |
|---|---|---|---|
| MAP | 70-110mmHg | 6.490 | **0**.**001** |
| cFLC | ≥57.2mg/L | 4.433 | **0**.**007** |

Multivariate logistic regression analysis using the clinical reference range cut offs. MAP and cFLC were independently associated with adverse CHD outcomes.

### Conclusions

The univariate analysis highlights that cFLC concentrations outside the normal reference range (>50mg/L) are predictive of adverse CVD outcomes in type II diabetes patients. This association is more significant when the optimal concentration for discriminating adverse outcomes (>57mg/L) is used as the categorical cut off. At this higher concentration, cFLC was independently associated with adverse CVD outcomes using multivariate analysis together with blood pressure measurements. These results indicate that cFLC measurements could have benefits for management of symptoms, risk stratification and monitoring disease progression type II diabetes patients.

## Claims

1. A method of determining a long-term prognosis in a subject identified as having diabetes, comprising detecting a total (i.e. combined λ and κ) amount of free light chains (FLC) in a sample from the subject, wherein the total amount of FLC in the sample is compared to a predetermined normal amount and a higher amount of FLC is associated with increased risk of a cardiovascular disease event or cardiovascular disease related death, wherein the sample is a sample of blood, serum or plasma and the subject does not present with symptoms of a B cell disease or chronic kidney disease (CKD), and the long term prognosis is at least 200 days from when the sample is taken.

2. A method according to claims 1, wherein the subject has type II diabetes.

3. A method according to claims 1 to 2, wherein the total FLC is determined by immunoassay using anti-free light chain antibodies or fragments thereof.

4. A method according to claim 3, wherein the antibodies are a mixture of anti-free κ light chain and anti-free λ light chain antibodies or fragments thereof.

5. A method according to claims 1 to 4, wherein the method comprises detecting the amount of FLC by nephelometry or turbidimetry.

## Patentansprüche

1. Verfahren zum Bestimmen einer Langzeitprognose bei einem Patienten, bei dem Diabetes festgestellt wurde, umfassend ein Erfassen einer Gesamtmenge (d.h. aus λ und κ kombinierten Menge) an freien Leichtketten (FLC) in einer Probe des Patienten, wobei die Gesamtmenge an FLC in der Probe mit einer vorbestimmten normalen Menge verglichen wird und eine höhere Menge an FLC erhöhtem Risiko eines kardiovaskulären Krankheitsereignisses oder durch kardiovaskuläre Erkrankung bedingten Todes zugeordnet ist, wobei die Probe eine Blut-, Serum- oder Plasmaprobe ist und der Patient keine Symptome einer B-Zell-Erkrankung oder chronischen Nierenerkrankung (CKD) aufweist und die Langzeitprognose mindestens 200 Tage ab Probenahme sind.

2. Verfahren gemäß Anspruch 1, bei dem der Patient Typ-II-Diabetes hat.

3. Verfahren gemäß Anspruch bits 2, bei dem die FLC-Gesamtmenge durch Immunassay mit Antikörpern gegen freie Leichtketten oder Fragmenten davon bestimmt wird.

4. Verfahren gemäß Anspruch 3, bei dem die Antikörper eine Mischung aus Antikörpern gegen freie κ-Leichtketten und Antikörpern gegen freie λ-Leichtketten oder Fragmenten daraus sind.

5. Verfahren gemäß Anspruch 1 bis 4, wobei das Verfahren ein Erfassen der Menge an FLC durch Nephelometrie oderTurbidimetrie umfasst.

## Revendications

1. Procédé permettant d'effectuer un pronostic à long terme chez un sujet identifié comme atteint de diabète, comprenant une étape consistant à détecter la quantité totale de chaînes légères libres FLC (c'est-à-dire de la combinaison de chaînes λ et de chaines κ) dans un échantillon du sujet, la quantité totale de FLC dans l'échantillon étant comparée à une quantité normale prédéfinie, et une quantité de FLC supérieure étant associée à un risque augmenté d'apparition d'une affection cardiovasculaire ou d'un décès lié à une affection cardiovasculaire, l'échantillon étant un échantillon de sang, de sérum ou de plasma et le sujet ne présentant pas de symptôme d'une affection des cellules B, ou d'une affection chronique des reins (CKD), et le pronostic à long terme étant d'au moins 200 jours à partir de la prise de l'échantillon.

2. Procédé conforme à la revendication 1,
selon lequel le sujet est atteint de diabète de type II.

3. Procédé conforme à la revendication 1 ou 2,
selon lequel la quantité totale de FLC est déterminé par un test immunologique en utilisant des anticorps anti-chaînes légères libres ou des fragments de tels anticorps.

4. Procédé conforme à la revendication 3,
selon lequel les anticorps sont un mélange d'anticorps anti-chaînes légères λ libres et d'anticorps anti-chaînes légères λ libres ou des fragments de tels anticorps.

5. Procédé conforme aux revendications 1 à 4,
comprenant une étape de détection de la quantité de FLC par néphélométrie ou par turbidimétrie.
